# EUROPEAN PATENT APPLICATION

(11) **EP 1 946 748 A1**
(43) Date of publication of application: **23.07.2008**
(21) Application number: 06807955.7
(22) Date of filing: 15.09.2006
(51) Int. Cl.: A61K 9/127, A61K 9/133, A61K 39/395, A61K 47/42, A61P 35/00, A61P 31/00

(54) **COMPOSITION FOR ADMINISTRATION TO MAMMALS OR HUMANS**

(30) Priority: 19.09.2005 ES 200502276
(71) Applicant: Serrano Gil, Daniel, 46220 Valencia (ES)
(72) Inventor: Serrano Gil, Daniel, 46220 Valencia (ES)
(86) International application number: PCT/ES2006/070135
(87) International publication number: WO 2007/034022

(57) **Abstract**

The invention relates to a composition for administration to mammals or humans, which is used to destroy damaged, pathogenic cells, including empty liposomes, structural protein elements and specific binding elements that are selected as a function of the pathogenic micro-organism or cell to be destroyed. When administered to the patient, the composition acts in the manner of an effective effector agent which interacts with the membrane of the target cell or with the membrane of the micro-organism in order produce discharge ports which release the intracellular content to the extracellular medium.

## Description

A COMPOSITION TO BE ADMINISTERED TO A MAMMAL OR TO A HUMAN BEING

INVENTION FIELD

The composition to be administered to a mammal or to a human being has its field of application as a therapeutic agent, as an effecting agent capable of interacting with an altered cell or a pathogenic micro-organism with the aim of disrupting the osmotic balance and achieving the release of the intracellular content into the extracellular medium.

It is intended as an antineoplastic agent in the treatment of cancer, being also applicable in the treatment of diseases caused by cells, bacteria, viruses, unicellular or multicellular organisms, in which their outermost covering is composed of a lipidic membrane, such as the cell membrane of eucaryotic cells, or those covering the aids virus (HIV).

PREVIOUS RELATED TECHNIQUE

With reference to cells, apoptosis refers to the active process, with morphological changes, in which cells are destined to die within a set period of time; the term necrosis refers to the violent and catastrophic processes that frequently appear as a consequence of exposure to toxins or of traumatic damage caused in the cell membrane with acute loss of cell function and regulation, resulting in an excessive osmotic process and ending in the death or lysis of the cell membrane, with the intracellular content being released into the extracellular medium.

Tumours appear following a succession of accumulative mutations that occur in a cell and are transmitted in the successive cell divisions. The rate of mutation depends on each tumour's genetic instability, and when a tumour is large enough to be clinically detected, the quantity of mutations is so high that the cells are spontaneously resistant to the action of some chemotherapy drugs. At the moment of diagnosing a tumour, the number of resistant cells is proportional to its size and its intrinsic mutation rate.

The tumour cell's resistance to chemotherapy is determined by the kinetics of their growth and by the appearance of spontaneous mutations. Although proliferative tumour cells are affected by the majority of chemotherapy agents, the effect on tumour cells with little activity is practically nil, and given the fact that the bulk of tumours are diagnosed in an advanced phase of their development, with a low growth fraction, they turn out not to be very sensitive to the action of the chemotherapy drugs.

The aim of chemotherapy treatments is to cause damage in cancerous cells, above all in the DNA, and to induce apoptosis. To do so it is necessary to ensure that as much active drug as possible reaches its molecular target inside the altered cell. Any external or internal circumstance which impedes or hampers this aim may be a cause of cell resistance.

Practically all the chemotherapy treatments based on cytostatic agents that have been seen to be affected *in vitro* by some resistance mechanism, penetrate into the cell by passive diffusion. Therefore, the higher the extracellular concentration of the drug, the greater is the quantity that penetrates into the cell and the greater its effectiveness.

Intracellular resistance mechanisms are those with the greatest influence on the failure of chemotherapy treatments. Thus, the reduction in the concentration of the drug at its target level is a result, among other causes, of it being expelled through the cell membrane by transport proteins, of the encapsulating of the chemotherapy drug molecules in cytoplasmic vesicles and variations in the transport between the nucleus and the cytoplasm, as well as of the alteration of the drug's intracellular metabolism by inactivation by oxidation and/or conjugation with glutation (GSH), and its subsequent expulsion from the cell by chemical pumps.

The majority of cytotoxic drugs used as antineoplastics cause alterations in the structure of the DNA which should induce cell apoptosis, however the increase in the DNA's repair capacity cancels out the effect of the drugs. Gene p53 should initiate cell apoptosis, however it has been shown that in many cancer cells this gene is mutated, or has even disappeared, with the result that although the altered cells have been damaged by the effect of the cytotoxic drugs, apoptosis fails to occur and the cells continue to divide.

Cytostatics are the antineoplastics most frequently used at present in chemotherapy and act by causing ruptures and/or alterations in the DNA of the cancer cells, which induce apoptosis and cell death with the aforementioned exceptions. Their relatively low specificity leads to side effects, since in addition to attacking the cancer cells they also destroy the cells of the gastric mucus and attack the cells of the immune system, leading to severe neutropenia in patients and leaving them defenceless against any opportunist infection or disease which can kill them.

New drugs, based on monoclonal antibodies and liposomes, have been developed in order to minimise the side effects in patients. The monoclonal antibodies in antineoplastic drugs can only be bound to a specific previously identified antigen in the cancer cells that need to be eliminated, therefore the active ingredient bound to the antibody will only affect the cancer cells that possess this antigen. With these, the side effects referred to above practically disappear.

Even more optimum is the use of pegylated liposomes with tumour specific monoclonal antibodies, onto which the antineoplastic drug's active ingredient is bound. Liposomes are vesicles made up of phospholipids with a composition that is very similar to that of the cell membrane, which enables the liposome to fuse with the cell membrane and ensures the penetration of the active ingredient contained in the liposome into the interior of the cell. With the use of medications administered by means of liposomes, the activity of the antineoplastic agent in the blood is maintained for a longer period of time, its administration is gradual, and the side effects in administered patients are even fewer. However, and despite improving quality of life and survival, this chemotherapy solution is expensive and its results modest against the resistance mechanisms of cancer cells, with the result that the public health system rarely uses them, and although in some cases the current chemotherapy is effective, for instance in child leukaemia, the majority of patients with metastasis and solid tumours die. Therefore there is a need to discover a new type of chemotherapy which avoids the mechanisms of resistance to current antineoplastics, a development which would mean salvation for millions of people worldwide.

OBJECT OF THE INVENTION

One object of the invention is to propose a composition to be administered to a mammal or a human being for the purpose of combating a disease caused by altered cells, bacteria, viruses or other organisms whose outer covering is a lipidic membrane.

Another object of the invention is a cancer treatment with a zero toxicity profile and a mechanism of action essentially different from that employed in conventional treatments.

SUMMARY OF THE INVENTION

Liposomes have also been used as structures without a classic active ingredient in numerous activities (antihypertensive, anti-inflammatory, etc.), although their use as empty liposomes without active ingredient against altered cells, especially cancer cells, is not known. Prior to this invention, the joint use of pegylated liposomes and monoclonal antibodies in the absence of antineoplastics has never been considered for use in the treatment of cancers and diseases produced by viruses, bacteria or others, and specifically in order to bring about the shedding of the intracellular content of an altered cell or of a micro-organism into the extracellular medium, its lysis and death.

Broadly speaking, the invention involves compositions and methods for treating the altered cells. More specifically, the invention involves compositions without chemotherapeutic content although made up of liposomes, monoclonal antibodies and structural proteins whose administration to a human being or to an animal with cell lesions entails cell opening, the shedding of the damaged cells' intracellular content into the extracellular medium and their lysis. In this way cell resistance mechanisms are avoided.

The present invention also provides a method for administering the composition to a human being or animal suffering from a cellular disease, such as a cancer - including carcinomas, leukaemias, myelomas, neuroblastomas, sarcomas, and lung, brain, ovarian, colon or breast cancers, while not being limited to these. In general this method consists of administering to the individual a quantity of the composition that represents an effective quantity capable of initiating the lytic process in the plasmatic membrane of the altered cells and, where appropriate, of the pathogenic micro-organisms. Effective quantities are determined following medical diagnosis.

The examples of the compositions which induce the cell opening include monoclonal antibodies specific to the disease being treated, bound to liposomes or vesicles that include structural proteins arranged on their external surfaces delimiting openings of a considerable size which, in the interaction of the liposomes with a damaged cell, remain open in order to induce plasmatic shedding into the extracellular medium, and the lysis and death of the damaged cell.

According to the invention, the monoclonal antibodies can be replaced by alcohols or glycoproteins that are also specific to the cell or micro-organism to be destroyed.

In consequence, a composition is provided to be administered to a mammal or a human being for the purpose of combating a disease caused by altered cells, bacteria, viruses or other organisms whose outer covering is a lipidic membrane.

It also provides a treatment against the disease having zero toxicity, and a mechanism of action that is essentially different to that employed in conventional treatments against damaged cells. The mechanism of action of the treatment proposed, having no chemical therapeutic agents, avoids the resistance mechanisms presented by the target cells, for instance cancer cells, unlike conventional antineoplastic agents.

The composition to be administered to a mammal or a human being is prepared, in a known manner, using liposomes, structural proteins and specific binding elements.

The administering of the composition to a mammal or a human being affected by the disease initiates the lytic process in the target cells or micro-organisms to be destroyed and the release of the intracellular content into the extracellular medium by way of the large openings initially made in lipidic membrane of the liposome by the protein and substantially maintained as drainage outlets following the interaction of the lipidic membrane with the plasmatic membrane of the target cells or micro-organisms at which its action is aimed.

More specifically, the present invention provides a composition for the treatment of cancer that includes a liposome that is preferably unilamellar, on whose structured a large opening has been defined with the integration of a structural protein, and its membrane incorporates antibodies specific to the damaged cell to be treated, so that the binding of the vesicles to the tumour's corresponding cell receptors will allow the fusion of the membranes of the vesicles and the specific cells. Once the binding of the liposomes to the target cells and the fusing of their membranes has occurred, the openings present on the liposomes form drainage outlets that connect the cytoplasm to the extracellular medium; the osmotic pressure is thus modified and the lytic process leading to cell death is initiated.

According to the invention, a composition designed to be administered to a mammal or to a human being, in order to destroy an altered or damaged cell or a pathogenic micro-organism, includes an empty unilamellar liposome whose structure presents an opening delimited by a structural protein element and whose membrane incorporates specific binding elements, for example selected monoclonal antibodies, where appropriate, depending on the cell to be destroyed, in such a way that on being administered to the patient the composition is an effective effecting agent capable of interacting with the membrane of the target cell or the membrane of the micro-organism, and on fusing with it, maintaining the opening in the liposome to a substantial degree with the aim of disrupting the osmotic balance of the cell and in addition ensuring that the opening is now used as a drainage outlet for releasing the intracellular content into the extracellular medium.

In substance, the empty unilamellar liposome present in the composition acquires a specific lipidic composition adapted to the target cell or micro-organism to be destroyed.

According to the invention, talin is preferred as the liposome's structural protein modifying element in the use of the intended composition.

The treatments based on the use of the proposed composition make use, as do conventional treatments, of monoclonal antibodies or other specific binding elements and liposomes, although without the disadvantages associated with the active ingredients incorporated into the latter, being harmless for the organism since they contain only phospholipids and proteins. In a neoplastic disease, the lethal effect of the composition on tumour cells is constituted by the opening and maintaining of a drainage outlet in the cell membrane which empties the latter of content following the interaction of the liposome associated with a structural protein, preferably talin, which generates a large opening in the liposome. The size of the opening can be previously selected and is in addition permanent in the liposome; consequently, in the fusion of the liposomes and the membranes of the damaged cells, an opening of substantially identical size is created and maintained in the cell membrane. Also the binding of multiple liposomes to the tumour cell membrane creates corresponding openings that will produce the immediate lysis of the cells and their death.

In the light of the above, the main advantages of the composition, compared with the antineoplastic treatments currently used, for administering to a mammal or to a human being in the treatment of altered cells, become evident. These include: a high specificity, with which the aim is to reduce or eliminate treatment side effects; the zero resistance offered by the membranes of the altered cells or those of the micro-organisms to the liposomic interaction proposed in comparison with chemotherapeutic compositions used up till now; its complete harmlessness, lacking as it does elements of a toxic or radioactive nature; its high selectivity with the use of specific binding elements, especially the use of monoclonal antibodies in the treatment of cancer, and its high lethal power when the liposome vesicles bind to the membranes of the pathogenic micro-organisms or to the membranes of the target cells.

The specific binding elements: monoclonal antibodies, glycoproteins or alcohols inserted into the membrane of the liposomes, whose action is to guide them to the micro-organism or target cell membranes, must be chosen selectively enough to cause the minimum of side effects.

## Claims

1. A composition designed to be administered to a mammal or to a human being, including at least one empty liposome in which at least one structural protein element is bound to the liposome, defining an opening of considerable size in the liposome membrane, and specific binding elements designed to ensure that the composition is an effecting agent capable of interacting with the membrane of the target cell or the membrane of the micro-organism, maintaining substantially the opening, with the aim of disrupting the osmotic balance and ensuring that the opening is a drainage outlet for releasing the intracellular content into the extracellular medium.

2. A composition intended to be administered to a mammal or to a human being, in accordance with Claim 1, designed so that the liposome is unilamellar and of a specific lipidic composition adapted to the cell or micro-organism to be combated.

3. A composition intended to be administered to a mammal or to a human being, in accordance with Claim 1, designed so that the structural protein element is talin.

4. A composition intended to be administered to a mammal or to a human being, in accordance with Claims 1 to 3, **characterised by** its use in the preparation of a treatment to combat altered cells and pathogenic micro-organisms.

5. A composition intended to be administered to a mammal or to a human being, in accordance with Claims 1 to 4, **characterised by** its use in the preparation of the treatment of a cancerous disease.

6. A composition intended to be administered to a mammal or to a human being, in accordance with Claims 1 to 4, **characterised by** its use in the treatment of a viral disease.
